# EUROPEAN PATENT SPECIFICATION

(11) **EP 3 890 812 B1**
(45) Date of publication and mention of the grant of the patent: **28.01.2026**
(21) Application number: 19813511.3
(22) Date of filing: 03.12.2019
(51) Int. Cl.: A61M 15/00, A61M 16/00, A61M 11/00, A61M 16/10

(54) **MOUTHPIECE AND NEBULIZER HAVING A MOUTHPIECE**
MUNDSTÜCK UND ZERSTÄUBER MIT EINEM MUNDSTÜCK
EMBOUT BUCCAL ET NÉBULISEUR COMPORTANT UN EMBOUT BUCCAL

(30) Priority: 04.12.2018 EP 18210195
(43) Date of publication of application: 13.10.2021
(73) Proprietor: PARI Pharma GmbH, 82319 Starnberg (DE)
(72) Inventor: KNOCH, Martin, 82467 Garmisch-Partenkirchen (DE); GALLEM, Thomas, 81371 Munich (DE); KAUFMANN, Mihaela, 85461 Kirchasch / Bockhorn (DE)
(74) Representative: Hoffmann Eitle
(86) International application number: PCT/EP2019/083441
(87) International publication number: WO 2020/115024

(56) References cited:
- EP-A1- 1 927 373
- EP-B1- 1 107 809
- EP-B1- 1 868 570
- US-A- 3 777 742
- US-A- 5 603 314
- US-A- 6 116 233
- US-A1- 2014 230 811

## Description

### TECHNICAL FIELD

The present disclosure relates to a mouthpiece for a nebulizer, particularly a handheld nebulizer. The concerned nebulizer (aerosol generator) is particularly used for aerosol therapy, wherein a liquid is nebulized/aerosolized by means of an aerosol generator, i.e. a vibratable membrane, for being administered to a user (patient).

### BACKGROUND

In use of such nebulizers, some nebulized liquid will be discharged from the nebulizer during exhalation of the user. In particular, known nebulizers of this type comprise an ambient opening and an exhalation opening each comprising a one way valve allowing that ambient air is drawn into the nebulizer during inhalation and allowing air to escape the nebulizer during exhalation. A nebulizer of this type is for example disclosed in EP 1 927 373 B1.

In some instances, the liquid to be nebulized/aerosolized may contain compounds which are detrimental for individuals staying in the environment in which the patient inhales even though they serve a therapeutic purpose with respect to the disease of the patient.

It is known in the art to use exhalation filters so as to avoid those components from being discharged (exhausted) into the environment. One example showing such an exhalation filter is EP 1 868 570 B1.

Figures 1 and 2 show a nebulizer 100 of an internal predevelopment similar to the one described in EP 1 927 373 B1. So as to provide an exhalation filter 30, it had first been conceived to connect a T-shaped adapter 120 at a connection port 121 to a chamber 105 of the nebulizer 100 for temporarily accommodating the aerosol generated by the aerosol generator 101. A mouthpiece 40 is connected to the adapter 120 at a boss 122 opposite to the connection port 121. Moreover, an exhalation filter 30 comprising a filter base 31, a filter top 33 detachably connected to the filter base 31 and a filter material 32 provided between the filter base 31 and the filter top 33 is provided. The filter base 31 is connected to a filter connection port 123 of the adapter 120 located between the connection port 121 and the boss 122.

This approach has however been conceived disadvantageous for several reasons. First of all, if the filter is directed upward (not shown), the filter is positioned closely in front of the user's nose and eyes. This is due to its height perceived uncomfortable, particularly if the user intends to read or watch television during inhalation.

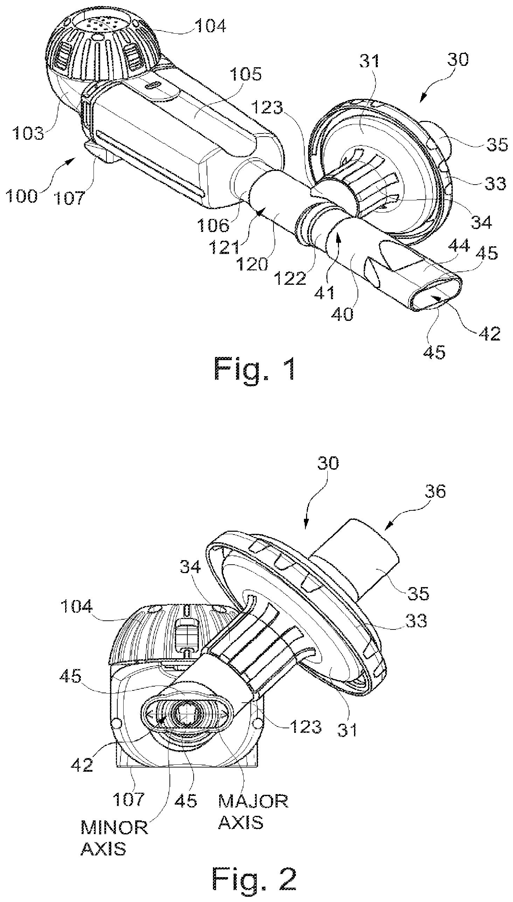

The attempt to rotate the filter to the right (alternatively to the left) as shown in figure 1 or so as to be directed downward leads to the problem that the nebulizer may no longer be placed on a horizontal surface because of the dimensions of the filter interfering with the horizontal surface. Positioning the filter in a tilted position, as shown in figure 2, leads to instability of the nebulizer when being placed on a horizontal surface. Thus, handling of the nebulizer is impaired.

Even further, this configuration employs a plurality of parts, namely the T-shaped adapter, the filter base, the filter material, the filter top and the mouthpiece (5 parts in total). This is perceived disadvantageous in handling the nebulizer as it needs to be disassembled for cleaning and subsequently again be assembled for use.

Moreover, this configuration significantly increases the overall length L_{O} of the nebulizer. However, such nebulizers are often used hands free by holding the nebulizer at the inhalation opening of the mouthpiece by means of the teeth. Yet, the longer the nebulizer is, the longer is the lever arm and the more difficult it is to use the nebulizer hands free. The same holds true if the filter is positioned in the tilted position as shown in figure 2 and if rotated to one side as shown in figure 1. These positions induce a rotational force on the teeth which is perceived uncomfortable.

Finally, this configuration leads to an increased dead space from the connection port 121 to the inhalation opening 42 at the mouthpiece 40 in which the aerosol may deposit and be washed out onto the filter during exhalation and, thus, be wasted.

Further prior art can be found in US 5 603 314 A and US 6 116 233 A. US 5 603 314 A discloses an improved filtration device suitable for reducing exhaled particles in an aerosol inhalation device. The filtration device includes a body portion having an interior chamber which communicates with an inlet and outlet opening through the chamber wall. Within the chamber is a substance recovery media designed to capture a portion of the exhaled particles from the patient and for assisting in returning a portion of the captured particles back to the patient, such as by a nebulizer. The filtration device further includes a filter layer located within the interior chamber for filtering out contaminants, such as viruses and bacteria, in the gaseous medium prior to releasing the remaining portion of the gaseous medium into the environment. Aerosol inhalation devices containing such filters are also provided by this invention.

US 6 116 233 A discloses an arrangement for delivering a drug aerosol comprises a sensor (3) which detects turbulent air flow during inhalation and causes the nebulizer (8,9,10,11,12,13) to generate the aerosol only during the inhalation phase of the breathing cycle. Airflow is diverted from the sensor during exhalation.

US 3 777 742 A discloses an apparatus for insufflating the airways of a patient with tantalum dust, either by the inhalation method or the catheter method.

US 2014/230811 A1 discloses a mouthpiece that is configured to be removably coupled with a nebulizer to deliver nebulized medicament to the airway of a subject. The mouthpiece is configured to connect to the nebulizer in a swappable manner with another mouthpiece that is configured to operate in a continuous use mode. By contrast, the mouthpiece is configured to operate in a breath assist mode, a breath activated or dosimetric mode, and/or in other modes that reduce medicament waste caused, for example, by exhalation of the subject back into the mouthpiece. This may be advantageous during the delivery some medicaments (e.g., steroids, antibiotics, and/or other medicaments). The mouthpiece is configured to maintain the double venturi effect that would be provided by the continuous use mouthpiece.

EP 1 107 809 A1 discloses an inhalator, comprising a manual apparatus with a handle body containing a receptacle in which a liquid medicament is atomized, and an aerosol chamber that is attached to the handle body and has a mouthpiece element through which the user suctions the aerosol when inhaling, wherein said aerosol is mixed with fresh air entering into the aerosol chamber via an inhalation orifice and exits through the mouthpiece element in a direction of flow. The aim of the invention is to embody said inhalator in such a way that the highest possible efficiency and low medicament loss is achieved. This problem is substantially solved in that an inhalation valve and an exhalation valve are provided which guide the inhalation air through the inhalation orifice and the exhalation air through the exhalation orifice, wherein the exhalation orifice is oriented in such a way that the exhalation air exits the exhalation orifice at an angle in relation to the direction of flow without mixing with the aerosol.

### SUMMARY

There is, hence, the need to provide a solution to alleviate the above problems and to provide a mouthpiece/nebulizer which is comfortable in use, easy to handle and/or having relatively little dead spaces.

This aim is according to an aspect achieved by a mouthpiece as defined in claim 1 as well as a nebulizer having such a mouthpiece as defined in claim 8.

Further embodiments may be derived from the dependent claims, the following description and the drawings. The invention is defined by the appended claims.

Accordingly, a mouthpiece for delivering an aerosol supplied by a nebulizer to a user and to be attached to a nebulizer is suggested. According to the present invention, the mouthpiece comprises a body defining a fluid path from an inlet port connectable to the nebulizer to an inhalation opening to be received in the mouth of a user. Moreover, an exhalation filter is integrated into the mouthpiece. In particular, the filter has a filter base in fluid communication with the fluid path, a filter top detachably connected to the filter base and a filter material provided between the filter base and the filter top. The filter top has an exhalation opening cooperating with a one-way valve allowing exhaustion of fluid from the fluid path through the filter material to the outside of the mouthpiece upon exhalation of a patient through the inhalation opening. In one embodiment, the one-way valve may be configured by a circular disk made of a flexible material, such as silicone or thermoplastic elastomer (TPE), covering the exhalation opening on a side opposite to the filter material. Accordingly, the disk is pushed away from the valve seat (such as ribs crossing (spanning) the exhalation opening) during exhalation so that air may escape from the mouthpiece. To the contrary, the disk is sucked and consequently pressed against the valve seat during inhalation so that no air may enter the mouthpiece during inhalation. To integrate the filter into the mouthpiece, the body of the mouthpiece and the filter base are an integrated one-piece unit. To put it differently, the body of the mouthpiece and the filter base are one element and are not detachable from each other. Due to this configuration, the tube connecting the body and the filter may be kept as short as possible. Therefore, the overall height of the mouthpiece can be reduced so that the filter does not form a visual obstruction for the user enabling him/her to e.g. read and watch television. In addition, the number of parts is significantly reduced as compared to the configuration as described with respect to figures 1 and 2, now consisting of three parts only, the integrated one-piece unit consisting of the body and the filter base, the filter material and the filter top. In addition, the length may be reduced, whereby dead spaces are minimized.

In a particular embodiment, the integrated one-piece unit is an injection molded part. Thus, the mouthpiece may be cost efficiently manufactured.

In addition, the overall length may be reduced in a mouthpiece as described above, whereby dead spaces are minimized. According to an aspect, this is assisted in that a distance between the center line of the filter base and the inhalation opening as seen in a side view is at least 30 mm or in another aspect at least 35 mm, but not more than 50mm, in another aspect not more than 40 mm. Thus, it may be ensured that there is still enough space between the filter base and the inhalation opening to accommodate the nose of a user without the nose touching the filter base but that the overall length of the mouthpiece is minimized.

The integration of the filter base into the body of the mouthpiece to form the integrated one-piece unit enables a reduction in the overall height of the mouthpiece to avoid a visual obstruction for the user during inhalation (see above). According to the present invention, the height of the mouthpiece as seen in a side view is not more than 90 mm, and according to another aspect not more than 85 mm, and in one particular embodiment less than 82 mm. For example, the maximum height may be 81.5 mm.

In one particular aspect, a retaining rib for being engaged behind the teeth of a user is provided on an upper side of the body and/or on a lower side of the body adjacent to the inhalation opening. Such a retaining rib facilitates hands free use of the nebulizer.

In order to even ergonomically improve the mouthpiece, one aspect suggests a front edge of the body surrounding the inhalation opening which is curved in a plan view and/or a side view. Thus, the inhalation opening is shaped like the mouth of a fish.

Moreover and according to one aspect the inlet port is conical to be force-fittingly connectable to a boss of the nebulizer (similar to a Luer taper). Such a configuration of the interface between the mouthpiece and the nebulizer in principle allows any orientation of the mouthpiece relative to the nebulizer, e.g. with the filter directed upward, downward, towards the sides or tilted. In combination with integrated one-piece unit, the orientation of the mouthpiece at the nebulizer may be advantageously determined by the configuration of the filter base and/or the configuration of the inhalation port of the mouthpiece despite this general possibility. For example, the filter base may be configured to interfere with the chamber of the nebulizer when the mouthpiece is rotated about the boss of the nebulizer preventing or limiting such rotation.

In another example, the inhalation opening is oval-shaped in a front view, the oval shape having a minor axis and a major axis, wherein the filter base (and/or the tube connecting the body and the filter base) extends from the body in a direction along the minor axis. Thus, only an orientation in which the filter is directed upward (or downward) is allowed.

According to a further aspect, a nebulizer for delivering an aerosol to a patient is suggested comprising an aerosol generator. The aerosol generator may be a so-called membrane aerosol generator comprising a membrane having a plurality of minute apertures in a central region. The liquid to be nebulized is applied to one side of the membrane in the central region and the membrane is vibrated by means of a vibrator (such as a piezoelectric element) typically at a frequency in the range from about 50 to about 300 kHz, specifically from about 60 to about 200 kHz, and more specifically from about 80 to about 180 kHz, or even more specifically from about 100 to about 140 kHz. Due to the vibration, the liquid applied to one side of the membrane passes the apertures and is nebulized on the opposite side of the membrane. The nebulizer further comprises a chamber for temporarily accommodating the aerosol generated by the aerosol generator and a mouthpiece as described above.

In one aspect, the aerosol generator, the chamber and the mouthpiece are arranged in that order along the longitudinal direction of the nebulizer. Thus, the nebulizer as such is already relatively long. Yet, by reducing the overall length of the mouthpiece along the longitudinal direction as described above, the overall length of the nebulizer may be minimized. Accordingly, the lever arm when holding the nebulizer with the teeth at the inhalation opening of the mouthpiece may be reduced facilitating hands free inhalation.

According to further aspect, the nebulizer further comprises a fluid reservoir or an interface configured to connect a fluid reservoir, wherein the aerosol generator comprises a vibratable membrane having a plurality of apertures (see above) and separating the fluid reservoir and the chamber.

When the fluid reservoir or the interface and the membrane are arranged in that order along the longitudinal direction of the nebulizer, the general length of the nebulizer is increased. Yet, it is possible to shorten the mouthpiece by integrating the filter base into the mouthpiece as explained above so that the overall length of the nebulizer can be minimized.

Similar applies to the case in which the chamber has a length along the longitudinal direction of the nebulizer between 30 mm and 100 mm, particularly 50 mm to 100 mm and more particularly not less than 70 mm, such as not less than 80 mm or not less than 90 mm. Again, the use of such a chamber which is beneficial for providing a sufficiently large bolus in the chamber which is to be inhaled by the user lengthens the nebulizer and, hence, the lever arm when the nebulizer is held by the teeth at the inhalation opening of the mouthpiece. Due to the possibility to shorten the mouthpiece including the filter this lever arm may again be reduced in length or minimized.

According to one aspect, the chamber has an ambient opening cooperating with a one-way valve allowing ambient air to enter the chamber from outside of the nebulizer upon inhalation of a patient through the inhalation opening of the mouthpiece.

According to a further aspect, the chamber has a volume of more than 45 ml, preferred more than 60 ml and even more preferred more than 90 ml.

The nebulizer may be used for diagnosis and therapy applications of liquids or fluids. The targeting area is the respiratory tract and especially the lungs and/or the nose. In the lung the central airways may be the targeting area. In many cases preferred for systemic or local applications the peripheral lung areas may be the targeting area.

The nebulizer system with the filter setup on the exhalation opening may be used in inhalation therapies, in which the user and/or patient nebulize liquids or fluids that may have a negative impact on the environment, for example other users, persons, patients, physicians, clinic personal, therapists, family members and/or animals.

The nebulizer may be used with fluids or liquids from the groups of immunosuppressive drugs, chemotherapeutic agents, radioactive compounds or tracers (e.g. for diagnosis) and/or opiates.

The nebulizer may be used with at least one fluid or liquid from the groups of immunosuppressive drug, chemotherapeutic agents, such as cytostatics, radioactive agents for diagnosis, Opioids, antiviral drugs, such as virustatics, antifungal drugs, such as antimycotics and gene therapeutic agents and/or derivates or mixtures thereof.

The nebulizer may be used with at least one fluid or liquid from the group of immunosuppressive drug. Immunosuppressive drugs or immunosuppressive agents or antirejection medications are drugs that inhibit or prevent activity of the immune system, for example to prevent graft rejection. The nebulizer may be used especially with fluid or liquids from the groups of immunosuppressive drug with severe side effects or adverse effects. In the group of agents acting as immunosuppressive drug may be used especially ciclosporin, tacrolimus, sirolimus, everolimus, mycophenolic acid, also called mycophenolate or mycophenolate-mofetil. Due to the severe side effects without medical indication exposition should be avoided.

The nebulizer may be used with chemotherapeutic agents, for example alkylating agents, antimetabolites, antimicrotubuli agents, cytoskeletal disruptors, histone deacetylase inhibitors, inhibitors of topoisomerase I + II, intercalating agents, kinase inhibitors, nucleotide analogs and precursor analogs and platinum-based agents.

As liquid or fluid may be used especially for the inhalation treatment following agents. For example may be used especially as alkylating agents for inhalation such as cyclophosphamide, or ifosfamide. For example may be used especially as topoisomerase I inhibitors for inhalation such as topotecan or irinotecan. For example may be used especially as topoisomerase II inhibitors for inhalation such as doxorubicin or etoposid. For example may be used especially as intercelating agents for inhalation such as anthracyclines, like doxorubicin. For example may be used especially as platinum-based agents for inhalation such as cisplatin, carboplatin, oxaliplatin, or satraplatin. Due to the severe toxicity without medical indication exposition should be avoided.

The nebulizer may be used with radioactive agents for diagnoses, for example technetium 99m [Tc99, Technegas, Technetium (99mTc), Technetium-99 (99Tc)], krypton (81mKr) inhalation gas, and Xenon-133 [Xenon Xe-133]. A number of isotopes, such as iodine-131 (131I), phosphorous-32 (32P), strontium-90 (90Sr), and yttrium-90 (90Y), may be used. Especially for a pulmonary ventilation and blood perfusion (V/Q) diagnose scan or scintigraphic pulmonary deposition studies the isotopes, krypton (81mKr) inhalation gas or technetium 99m (99mTc), may be used. Exposition of medical staff should be avoided.

The nebulizer may be used with opioids, for example endogenous opioids, opium alkaloids and derivatives, and synthetic opioids. Exposition of small children and babies may be avoided.

In another embodiment of the nebulizer, the nebulizer may be used with fluids or liquids of the groups of antiviral drugs, such as for example aciclovir or ganciclovir.

In another embodiment of the nebulizer, the nebulizer may be used with fluids or liquids of the groups of an antifungal medication, also known as an antimycotics, is a pharmaceutical fungicide or fungistatic. For example may be used especially amphotericin B or variconazole, which may have severe side effects.

The combination of inhalative antivirals and inhalative antifungal medication may have severe side effects and can result in clinical admission of user or patient.

In a further embodiment of the nebulizer, the nebulizer may be used with fluids or liquids of the groups of viral gene therapy agents or non-viral gene therapy agents. The transferred nucleotide constructs may be single or double stranded DNA, RNA, or siRNA. In one study the gene therapeutic agent carries especially the CF gene to substitute and cure the cystic fibrosis deficiency. For the transfer to the patient the substitute is integrated in a viral vector and masked in liposomes. The, from UK CF Gene Therapy Consortium (GTC) called, inhalative gene therapeutic agent "pGM169/GL67A" is under clinical evaluation.

Also mixtures of fluids and liquids may be used with the nebulizer, especially mixtures of the mentioned fluids and liquids above.

As previously indicated, the mouthpiece and the nebulizer described herein may be used with several different liquids or fluids to be nebulized. One particular example concerns a pharmaceutical composition comprising an inhalable immunosuppressive macrocyclic active ingredient for use in the prevention or treatment of a pulmonary disease or condition by inhalation.

The pulmonary disease or condition may be selected from asthma, refractory asthma, chronic obstructive bronchitis, parenchymal, fibrotic and interstitial lung diseases and inflammations, bronchiolitis obliterans (BOS), and acute and chronic organ transplant rejection reactions after lung transplantation and the diseases resulting therefrom.

The pulmonary disease or condition may be bronchiolitis obliterans (BOS), optionally after acute and chronic organ transplant rejection reactions after lung transplantation or after hematopoietic stem cell transplantation (HSCT).

The inhalable immunosuppressive macrocyclic active ingredient may be selected from cyclosporine A (CsA), tacrolimus, sirolimus and/or everolimus, preferably in a therapeutically effective amount. An immunosuppressive compound as named above may be present as the only active ingredient or in form of a mixture of two or more different inhalable immunosuppressive macrocyclic active ingredients, optionally in combination with other non-immunosuppressive and/or non-macrocyclic active ingredients. In specific embodiments, however, the pharmaceutical compositions for use according to the present invention comprise just one inhalable immunosuppressive macrocyclic active ingredient.

The inhalable immunosuppressive active ingredient may be cyclosporine A (CsA).

The cyclosporine A may at least partly be present in liposomally solubilized form (L-CsA).

The inhalable immunosuppressive active ingredient may be tacrolimus.

The tacrolimus may at least partly be present in liposomally solubilized form (L-CsA).

The inhalable immunosuppressive active ingredient may be a combination of cyclosporine A (CsA) and tacrolimus.

The combination of cyclosporine A (CsA) and tacrolimus may at least partly be present in liposomally solubilized form (L-CsA).

The aerosol may be generated by nebulization of a liquid composition comprising the inhalable immunosuppressive macrocyclic active ingredient, particularly cyclosporine A (CsA).

Furthermore, especially in view of the fact that a macrocyclic immunosuppressant, such as cyclosporine A, should be administered in the minimal amount possible and exclusively or predominantly to the targeted tissues, there is still need for a pharmaceutical composition comprising a macrocyclic immunosuppressant for inhalation purposes that allows for an effective delivery of the chosen immunosuppressive wherein as much as possible of the administered immunosuppressive is actually delivered to the targeted tissues and a minimized amount of the compound is exhaled by the patient during administration. Furthermore, there is still a need for an improved transport of macrocyclic immunosuppressant especially to the peripheral tissues of the lungs.

Thus, and independent of the unitary one-piece configuration of the mouthpiece described above, it may be beneficial to use a nebulizer for nebulizing a fluid, particularly for administering the macrocyclic immunosuppressant such as that described above, the nebulizer having:
an aerosol generator, wherein the aerosol generator comprises
   a fluid reservoir for holding the fluid to be nebulized and to supply the fluid to a vibratable membrane or an interface configured to connect such fluid reservoir;
   the vibratable membrane having a plurality of apertures, the apertures being adapted to produce an aerosol comprising fluid droplets having a mass median aerodynamic diameter (MMAD) of less than about 4.0 µm as measured with a 0.9 % (w/v) aqueous solution of sodium chloride; and
a chamber for temporarily accommodating the aerosol generated by the aerosol generator, the chamber having an inner lumen with a volume in the range from about 50 to about 150 ml.

The nebulizer may have a mouthpiece for delivering the aerosol supplied by the nebulizer to the user or patient. The mouthpiece may have an exhalation filter and may be configured as described above.

In one embodiment, the vibratable membrane has from about 30 to about 250 apertures per mm².

Further, the plurality of apertures of the vibratable membrane may have a tapered shape narrowing towards the aerosol release side of the vibratable membrane.

Furthermore, the apertures of the vibratable membrane may have an exit diameter in the range from about 1.5 µm to about 3.0 µm as measured by scanning electron microscopy (SEM).

Moreover, the aerosol produced by the aerosol generator may comprise droplets, wherein at least 50%, more specifically from about 60 to about 95% or more specifically from about 70% to about 90% of the total volume or mass of droplets have a diameter of less than about 5 µm (as measured by laser diffraction 'MMD' or by a multistage cascade impactor 'MMAD' as described in detail below) with a liquid composition comprising L-CsA in a concentration of about 4 mg/mL.

In one example, the fluid is administered to the user or patient at a total output rate (TOR) of at least 200 mg/min, more specifically at a total output rate in the range from about 200 to about 250 mg/min.

Further features and aspects are described with respect to particular embodiments making reference to the accompanying drawings.

### BRIEF DESCRIPTION OF THE DRAWINGS

In the drawings:
Figure 1 shows a perspective view of a nebulizer in an intermediate stage of the development (pre-development) with an exhalation filter attached to the nebulizer via a T-shaped adapter, wherein the filter is directed to the right in front view and a separate mouthpiece is connected to the adapter.
Figure 2 shows a perspective front view on the nebulizer of figure 1, but with the filter being tilted to the right.
Figure 3 shows a perspective side view of a mouthpiece according to an aspect.
Figure 4 shows an exploded view of the mouthpiece of figure 3 and a nebulizer mixing chamber.
Figure 5 shows a longitudinal sectional view of the nebulizer and the mouthpiece of figures 3 and 4.

### DETAILED DESCRIPTION

In the several drawings, the same reference numerals have been used for the same and the like elements.

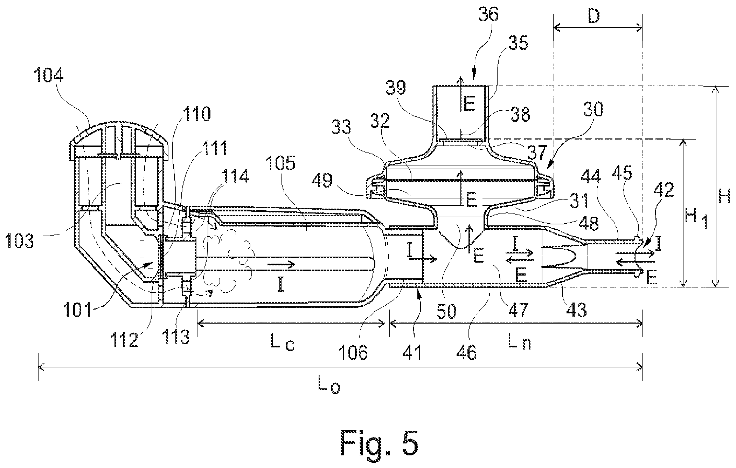

The drawings show a nebulizer 100 comprising an aerosol generator 101 (see figure 5) and a chamber 102 for temporarily accommodating the aerosol generated by the aerosol generator.

In the example, the aerosol generator 100 comprises a membrane 110 having a plurality of apertures in a central region 111. Moreover, a piezoelectric ring 112 is provided to vibrate the membrane 110.

The apertures may be adapted to produce an aerosol comprising droplets having a mass median aerodynamic diameter (MMAD) typically in the range of less than about 5 µm or of less than about 4.0 µm as measured by nebulization of a 0.9 % (w/v) aqueous sodium chloride solution. In specific embodiments, the plurality of apertures of the vibratable membrane are adapted to produce an aerosol comprising droplets having a mass median aerodynamic diameter (MMAD) in the range from about 1.5 µm to about 5.0 µm, such as from about 1.5 µm to about 4.0 µm or from about 2.0 µm to about 4.0 µm or from 2.4 µm to about 4.0 µm as measured by nebulization of a 0.9 % (w/v) aqueous sodium chloride solution.

In further specific embodiments, the plurality of apertures are adapted to produce an aerosol comprising droplets having a mass median aerodynamic diameter (MMAD) in the range from about 1.5 µm to about 3.9 µm, such as from 2.0 µm to about 3.9 µm or from 2.4 µm to about 3.9 µm as measured by nebulization of a 0.9 % (w/v) aqueous sodium chloride solution.

In alternative embodiments, the mass median aerodynamic diameter (MMAD) of the droplets generated by the vibratable membrane comprising the plurality of apertures adapted accordingly can be determined by the nebulization or aerosolization of a liquid formulation of liposomally solubilized CsA (L-CsA) with a CsA-concentration of 4.0 mg/mL as described in Example 2.2 below. According to these embodiments, the plurality of apertures are adapted to produce an aerosol comprising droplets having a mass median aerodynamic diameter (MMAD) typically below about 6 µm, such as in the range of from about 2.0 µm to about 5.5 µm, or from 2.5 µm to about 4.5 µm or from about 2.8 µm to about 4.4 µm. The values for the mass median aerodynamic diameter (MMAD) as referred to herein typically may be associated with a Geometric Standard Deviation (GSD) in the range of 1.1 up to 2.4, more specifically of 1.2 up to 2.2 or of 1.3 up to 2.0 or more specifically of lower than 2.0 such as 1.4 up to 1.8 or up to 1.7. In cases in which the GSD value is below 2.0 the corresponding droplet size distribution generated by the aerosol generator or the vibratable membrane, respectively, is referred to as a narrow droplet size distribution.

The mass median aerodynamic diameter and other metrics of the aerosolized pharmaceutical compositions can be determined by methods known to those of skill in the art by means of e.g. an impactor such as a cascade impactor or by laser diffraction analysis as described e.g. in Eur. Ph. 2.9.44 or to USP chapter <1601>. As a cascade impactor a multistage cascade impactor may be used such as an 'Anderson Cascade Impactor' (ACI) or preferably a 'Next Generation Impactor (NGI). These methods allow for the determination of several metrics of the generated aerosol comprising the present pharmaceutical compositions, such as the MMAD as mentioned above, the fine particle dose or fraction (FPD or FPF), the geometric standard deviation (GSD), as well as the respirable dose or fraction (RD or RF).

The use of a multistage cascade impactor such as the ACI or preferably the NGI allows for the characterization of the aerodynamic metrics e.g. generated by the aerosolization/nebulization of a 0.9 % (w/v) solution of NaCl as well as for the aerosolization/nebulization of a liquid pharmaceutical composition.

The vibratable membrane (110) may typically comprise from about 1000 to about 5000 apertures, or from about 2000 to about 4000 apertures, often from about 1500 to about 3500 apertures. Based on a typical perforated surface area of such a membrane in the range less than about 30mm², preferred from about 5 mm² to about 30 mm², more preferred from about 6 mm² to about 20 mm² and even more preferred from about 7 mm² to about 15 mm², the vibratable membrane typically may have from about 30 to about 700 apertures per mm², often from about 60 to about 600 or from about 80 to about 500 or from about 100 to about 400 apertures per mm² of the perforated surface area.

The mean geometrical diameter of the apertures as measured by scanning electron microscopy (SEM) typically may be less than 4.0 µm or, more specifically in the range of from about 1.5 µm to about 3.0 µm, or from about 1.6 µm to about 2.8 µm or from about 1.8 µm to about 2.6 µm with a standard deviation of typically +/- 0.4 µm or more specifically of +/- 0.3 µm or even +/-0.2 µm.

In specific embodiments, the plurality of apertures of the vibratable membrane or, more specifically, each aperture of the plurality of apertures may have a tapered shape narrowing towards the aerosol release side of the vibratable membrane. Accordingly, the aperture may be formed as a channel which continuously or discontinuously narrows towards the aerosol release side of the vibratable membrane.

In further specific embodiments, the apertures or a single aperture of the plurality of apertures of the vibratable membrane accordingly may have an exit diameter corresponding to the minimal mean geometrical diameter of the apertures as described above.

In addition, the nebulizer comprises a fluid reservoir 103 applying a liquid to one side of the central region 111 of the membrane 110 containing the apertures (see figure 5). The fluid reservoir 103 is closed by a lid 104. In general, such a nebulizer is disclosed in EP 1 927 373 B1 and EP 1 353 759 B1. Instead of a fluid reservoir it is also conceivable to provide an interface such as a needle or collar configured to connect a fluid reservoir such as an ampoule. Such system is for example disclosed in EP 1 919 542 B1.

In use, a liquid applied to the one side of the membrane 110 (aerosol generator) is passed through the apertures by vibrating the membrane via the piezoelectric element 112 whereby the aerosol is introduced (ejected) into the chamber 105. Thus, the membrane 110 (aerosol generator) is disposed between the fluid reservoir 103 and the chamber 105. The chamber 105 is configured for temporarily accommodating the aerosol generated by the membrane 110 as a bolus to be inhaled by a user.

The chamber has an inner lumen with a volume in the range from about 50 to about 150 mL. In specific embodiments, the inner lumen of the chamber (105) may have a volume in the range from about 70 to about 130 mL or in the range from about 75 to about 125 mL or more specifically in the range from about 80 to about 120 mL and even more specifically in the range from about 90 to about 110 mL or to about 100 mL.

A longitudinal end of the chamber 105 opposite to the end at which the membrane 110 is disposed comprises a boss 106 defining a discharge opening and being circular in cross section. The boss 106 may be cylindrical or tapered resembling a male taper similar to a Luer taper system.

Further, the chamber 105 resembles a stand enabling to place the nebulizer 100 on a horizontal surface such as a table.

In addition, ambient openings 113 are disposed at one and of the chamber 105 close to the aerosol generator 101. These openings 113 cooperate with a one-way valve 114 such as flaps or reed valves allowing ambient air to enter the chamber 105 upon inhalation of a patient.

If the nebulizer 100 is to be used with a liquid containing a compound that may be detrimental to individuals staying in the environment in which the user inhales, there is a need to implement an exhalation filter 30.

In the predevelopment it was conceived to use a T-shaped adapter 120 to attach the exhalation filter 30.

The T-shaped adapter 120 comprises an inlet port 121 being circular in cross section. The inlet port 121 is cylindrically shaped or tapered resembling a female taper similar to a Luer taper. The T-shaped adapter 120 is connected by force fit with its inlet port 121 to the boss 106.

An outlet port 122 is disposed opposite to the inlet port 121. The outlet port 122 is configured similar to the boss 106 and is, hence, also circular in cross section and may be cylindrically shaped or tapered resembling a male taper of a Luer taper system.

A filter connection port 123 is disposed at an intermediate position between the inlet port 121 and the outlet port 122 and extends substantially perpendicular to a direction from the inlet port 121 to the outlet port 122 defining the T-shape. The filter connection port 123 is circular in cross section and may be cylindrically shaped or tapered resembling a male taper of a Luer taper system.

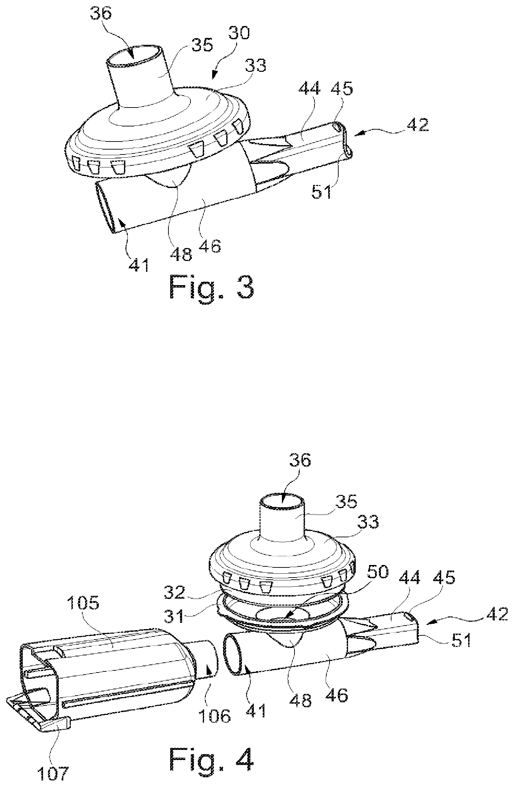

The exhalation filter 30 comprises a filter base 31, a filter material 32 (see figures 4 and 5) and a filter top 33.

The filter base 31 comprises a connecting tube 34 which is circular in cross section and may either be cylindrically shaped or tapered resembling a female Luer taper. Accordingly, the exhalation filter 30 may be connected to the filter connecting port 123 by force fit.

The filter material 32 may be made of polypropylene or the like. In one example, the filter may be made of blended synthetic fibres for example attached to polypopylene spun bonded scrim. The filter may typically have a thickness of between 1.5 mm to 5.5 mm or 2 mm to 5 mm or about 3.5 mmm. The air permeability at 200 Pa may be from about 400 to about 600 L/m²s and/or an air flow resistance may be about 46.0 PA at a medium velocity of 9.5 m/min. An exemplary filter is Microstat + 250 MED sold by Riensch & Held, Germany). The filter material 32 is sandwiched between the filter base 31 and the filter top 33 as will be apparent from figure 5.

Thus, the filter base 31 of the filter top 33 form a compartment accommodating the filter material 32.

The filter top 33 is detachably mounted to the filter base 31 using for example a snap fit. The filter material may be changed by detaching the filter top 33 from the filter base 31.

Moreover, the filter top may comprise another boss 35 allowing connecting a tube or other equipment. Yet, this boss 35 may as well be omitted for example to further reduce the device height.

Further, an exhalation opening 36 is provided in the filter top 33. The exhalation opening 36 may be circular in cross section. In one aspect, a plurality of radial ribs 37 (figure 5) extend across the exhalation opening 36 and a pin 38 is formed in a center thereof. A one-way valve 39 (in the present aspect a disk made of a flexible material) is fixed to the pin 38 so as to be disposed on a side of the ribs 37 opposite to the compartment and the filter material 32. Thus, the one-way valve 39 allows fluid to flow from the compartment between the filter base 31 and the filter top 33 valve 39 opens (the disc 39 lifts from the radial ribs 37 allowing the fluid to pass between the ribs 37 through the exhalation opening 36 to the outside). However, the valve 39 blocks the flow of fluid in the opposite direction from the environment toward the compartment in that it closes (the disc 39 is pressed against the radial ribs 37 closing the openings between the ribs 37 and hence blocking the exhalation opening 36).

Moreover, figures 1 and 2 show a mouthpiece 40 having a connecting portion 41 circular in cross section and cylindrically shaped or tapered resembling a female Luer taper, like a tube connector used in intensive care units. The connecting portion 41 is connected to the outlet port 122 of the T-shaped adapter 120 by force fit. This may provide an airtight seal at the connection between the connecting port 41 and the T-shaped adapter 120.

An inhalation opening 42 may be provided opposite to the connecting portion 41. The inhalation opening 42 is oval in cross section as best visible from figure 2. Thus, the inhalation opening 42 comprises a major axis substantially oriented horizontally and/or parallel to the stand 107 and a minor axis extending perpendicularly to the major axis.

In this context, the mouthpiece 40 contains a taper 43 in which the mouthpiece 40 changes its cross section from a circular cross-section at the connecting portion 41 to the oval cross-section of the inhalation opening 42. Thus, the mouthpiece 40 tapers at an intermediate position 43 defining a substantially flat portion 44 to be accommodated in the mouth of a user or patient.

Further, retaining ribs 45 are formed on the long sides of the oval cross-section adjacent the inhalation opening 42 extending substantially perpendicular from the portion 44. Thus, the teeth may catch or engage behind the retaining ribs 45 and the user may hold the entire nebulizer with his/her teeth and use it hands free.

This predevelopment, even though providing the required function of filtering exhausted air, has, however, disadvantages.

For example, if the filter 30 is directed upward (not shown), the filter is positioned closely in front of the user's nose and eyes. In other words, the height H of the nebulizer is too large. This is perceived uncomfortable, particularly if the user intends to read or watch television during inhalation.

The attempt to rotate the filter to the right (alternatively to the left) as shown in figure 1 or so as to be directed downward leads to the problem that the nebulizer may no longer be placed on a horizontal surface in view of the stand 107 because of the dimensions of the filter. Positioning the filter in a tilted position, as shown in figure 2, leads to instability of the nebulizer when being placed on a horizontal surface. In particular in this configuration, the nebulizer would tend to tilt over. Thus, handling of the nebulizer is impaired.

Even further, this configuration employs a plurality of parts, namely the T-shaped adapter 120, the filter base 31, the filter material 32, the filter top 33 and the mouthpiece 40 (making in total 5 parts). This is perceived disadvantageous in handling the nebulizer as it needs to be disassembled for cleaning and subsequently again be assembled for use and may lead to an inaccurate assembly of parts and to misuse without filter and exhalation valve.

Moreover, this configuration significantly increases the overall length L_{O} of the nebulizer. However, such nebulizers are often used hands free as explained above by holding the nebulizer at the inhalation opening of the mouthpiece using the retaining ribs 45 by means of the teeth. Yet, the longer the nebulizer is, the longer is the lever arm and the more difficult it is to use the nebulizer hands free. This effect is even more severe when a relatively long mixing chamber 105 having a length L_{C} between 80mm and 100 mm is used.

Finally, this configuration leads to an increased dead space from the connection port 121 to the inhalation opening 42 at the mouthpiece 40 in which the aerosol may deposit and be washed out onto the filter 32 during exhalation and, thus, be wasted.

Most of these problems have been alleviated by the present disclosure. In particular, the adapter 120 and the filter base 31 have been integrated into the mouthpiece 40 as particularly shown in figures 3 to 5 as one-piece unit with a defined connector to the camber 105.

Thus, the mouthpiece 40 according to an aspect comprises a body 46 having the inlet port 41 resembling the connection port 121 of the adapter 120. The inlet port 41 is, thus, connectable to the boss 106 of the chamber 105 of the nebulizer 100 by force fit.

The body 46 defines a fluid path 47 from the inlet port 41 to the inhalation opening 42.

In addition, the filter base 31 is integrally formed with the body 46. In particular, the body 126 comprises a nipple 48 perpendicularly extending from the body 46 in a direction of the minor axis of the inhalation opening 42. The nipple 48 resembles the filter connection boss 123 and connection port 35. Thus, the body together with the exhalation filter 30 forms a further fluid path 49 from an opening 50 opening into the fluid path 47 through the exhalation filter 30 passing the filter material 32 and exiting through the exhalation opening 36.

Thus, the filter base 31, the nipple 48 and the body 46 including the inhalation opening 42 and the connection port 41 define an integrated one-piece unit. In one aspect this integrated one-piece unit is formed by injection molding.

The filter material 32 and the filter top 33 may remain the same as previously described.

Similar applies to the configuration of the mouthpiece with respect to the inhalation opening 42, the ribs 45, the tapering 43 and the portion 44.

However and in order to even further improve the ergonomic shape of the mouthpiece 40 adjacent to the inhalation opening 42, the edge 51 bordering the inhalation opening 42 is shaped similar to the mouth of a fish. To put it differently, the edge is curved inwardly in a side view and outwardly in a top view as best visible from figure 3.

In a further embodiment the opening 42 may have at least two ribs juxtaposed to each other in the longitudinal direction L to ensure a better hold with the teeth.

In use, a liquid 13 is nebulized by the aerosol generator 101 into the chamber 105 were the aerosol is temporarily stored. Upon inhalation of a user through the inhalation opening 42, ambient air flows through the openings 113, wherein the valves 114 open into the chamber 105 and entrains the aerosol, which flows from the chamber 105 through the boss 106 and the connection port 41 through the fluid path 47 and is inhaled through the inhalation opening 42 as indicated by the arrows I in figure 5. During inhalation, the disk 39 closes the openings between the radial ribs 37 and, hence, the exhalation opening 36 so that no ambient air may enter through the exhalation opening 36.

Upon exhalation the exhaled air from the patient or user is introduced through the inhalation opening 42 and flows through the fluid paths 47 and 49 as indicated by the arrow E in figure 5 through the opening 50 into the exhalation filter 30 passes the filter material 32 and exits the inhalation filter 30 through the exhalation opening 36. During this process, the disk 39 lifts from the radial ribs 37 and allows the exhaled and filtered air to pass and exit through the exhalation opening 36. During this process, the valves 14 close the openings 113 so that the only possibility for the exhaled air to escape is through the filter 30.

Especially fluids that are partly toxic should be avoided to be set out to the environment for assistant persons.

Due to the integration of the filter base 31 and the adapter 120 into the mouthpiece 40, particularly into the body 46 thereof, the length L_{M} of the mouthpiece 40 can be kept short as compared to the length of the adapter 120 and the mouthpiece 40 shown in figures 1 and 2. Accordingly, the overall length L_{O} of the nebulizer 100 can be reduced. As a result, the lever arm can be shortened and it is more easy to use the nebulizer 100 hands free by retaining the nebulizer 100 via the teeth engaging with the retaining ribs 45 and thereby support the nebulizer 100.

The setup as a one-pieced unit, of the filter base 31, the nipple 48 and the body 46 including the inhalation opening 42 and the connection port 41, ensures that the entire exhaled air including the generated aerosol is filtered before the aerosol is exhausted from the device.

In addition, by keeping a distance D between the filter base 131 and inhalation opening 42 as seen in the side view (figure 5) between 30 and 50 millimeters, preferably between 35 millimeters and 40 millimeters, there is sufficient space to accommodate the nose of a user without the nose touching the inhalation filter 30 at the same time reducing the overall length L_{M} of the mouthpiece 42 to its minimum.

In addition, because of the incorporation of the filter connecting port 123 and the connecting tube 34 into the body 46, the filter base 31 may be brought closer to the body 46 whereby the overall height H in side view may be reduced to not more than 90 mm. In the shown example, the maximum height H is 81.5 mm. When omitting the boss 35, the height may even be further reduced to the height H₁. The overall height from the filter base to the exhalation filter opening of the mouthpiece will be further reducted. As one aspect, the height of the mouthpiece as seen in a side view (Figure 5) is not more than 75 mm, and according to another aspect not more than 70 mm, and in one particular embodiment less than 57 mm. For example, the maximum height may be 56.2 mm.

As a result, the suggested aspects provide significant advantages particularly as compared to the internal predevelopment.

The inlet port 41 has been described above as forming a female taper of a Luer taper (the boss 106) cooperating with a male taper (the boss 106). Yet, the inlet port 41 may as well be configured as a male taper of a Luer taper cooperating with a female taper at the chamber 105.

The nebulizer and mouthpiece as described in detail above allows for the administration of various pharmaceutical compositions, particularly those mentioned above. Especially when provided in form of a solution, colloidal formulation or suspension, the nebulizer and mouthpiece allow for generating the aerosol comprising the pharmaceutical compositions to be administered in a high fraction of droplets or particles which are able to reach the periphery of the lungs ("Fine Particle Fraction"; FPF). In specific embodiments, these droplets or particles have a mass median particle diameter of equal or lower than 5 µm as measured e.g. by laser diffraction using a Malvern MasterSizer X or using a multistage cascade impactor such as the Anderson Cascade Impactor (ACI) or the Next Generation Impactor (NGI).

In specific embodiments, the aerosol to be administered to the patient comprises droplets wherein at least 50%, more specifically from about about 60 to about 95% or more specifically from about 70% to about 90% of the total number of droplets have a diameter of less than 5 µm (as measured by laser diffraction or by a multistage cascade impactor as described above) when measured with a liquid composition comprising L-CsA in concentration of 4 mg/mL as described in Example 1 below.

The nebulizer and mouthpiece as described in detail above further allows for the administration of pharmaceutical compositions with a higher percentage of drug available as delivered dose (DD) and respirable dose (RD) compared to conventional nebulizers such as jet nebulizers. The term 'delivered dose' (DD) as used herein means the fraction of the aerosolized active ingredient collected on an inspiratory filter during standardized simulated breathing, i. e. the percentage of drug filled into the nebulizer for aerosolization and inhalation which is actually delivered to the airways. Accordingly, in specific embodiments the inhalable immunosuppressive macrocyclic active ingredient, preferably cyclosporine A (CsA) is delivered to the lungs (or the lung) of the subject in an amount of at least 60% or even at least 70%, more specifically in an amount in the range from about 70% to about 80% of the amount filled into the nebulizer during standardized simulated breathing, which measure the amount, which is normally administered to the subject.

Furthermore, the nebulizer and mouthpiece as described above allows for the administration of the pharmaceutical compositions at a high total output rate (TOR) which is typically above about 150 mg/min corresponding to about 0.15 mL/min for liquid compositions with a relative density of about 1, with regard to the final pharmaceutical composition to be nebulized and administered. In specific embodiments, the inhalable immunosuppressive macrocyclic active ingredient is administered to the subject at a total output rate (TOR) of at least 200 mg/min or more specifically at a total output rate in the range from about 200 mg/min to about 300 mg/mL or from about 200 to about 250 mg/min. Accordingly, the nebulizer and mouthpiece as described above allows for a short nebulization time of the present liquid pharmaceutical composition. Obviously, the nebulization time will depend on the volume of the composition which is to be aerosolized and on the output rate.

The volume of a unit dose of the pharmaceutical compositions is preferably low in order to allow short nebulization times. The volume, also referred to as the volume of a dose, or a dose unit volume, or a unit dose volume, should be understood as the volume which is intended for being used for one single administration. A unit dose is defined as the dose of cyclosporine A in the formulation filled in the nebulizer for one single administration. Specifically, the volume of a unit dose may be less than 10 mL or less. Preferably, the dose unit volume is in the range from about 0.3 to about 3.5 mL, more preferably about 1 to about 3 mL. For example, the volume is about 1.25 mL or about 2.5 mL. In case the formulation is obtained after reconstitution, the volume of the saline solution for reconstitution should be adapted according to the desired volume of the reconstituted formulation.

The unit dose of the macrocyclic immunosuppressive active ingredient, preferably CsA typically is within the range of from about 1 mg to about 15 mg. In specific embodiments, a unit dose of the chosen active ingredient, preferably CsA is about 5 mg or about 10 mg.

Accordingly, in specific embodiments, 1 mL of the pharmaceutical composition comprising an inhalable immunosuppressive macrocyclic active ingredient is aerosolized (nebulized) within a period of up to about 5 min, preferably of up to about 4 min, specifically in cases in which a liquid composition comprising CsA in liposomally solubilized form at a concentration of 4 mg/mL is administered.

In addition to providing a high delivered dose and having short nebulization times, the nebulizer and mouthpiece for administering pharmaceutical compositions is constructed in such way that contamination of the environment with the immunosuppressive macrocyclic active ingredient such as CsA or tacrolimus is prevented by the exhalation filter of the mouthpiece. Such exhalation filter may reduce or avoid emission of the exhaled amount of the macrocyclic immunosuppressive active ingredient such as CsA or tacrolimus to the environment. However, due to the high percentage of the droplets of the aerosol actually delivered to the airways as described above, the nebulizer and mouthpiece as described above allow for the significant reduction of the exhaled active ingredient. Accordingly, in specific embodiments of the present invention the amount of the inhalable immunosuppressive macrocyclic active ingredient exhaled by the subject is up to 10%, more specifically from about 4% to about 8% of the total amount of active ingredient filled into the nebulizer during standardized simulated breathing, which measure the amount, which is normally administered to the subject and could be collected on the exhalation filter.

The pharmaceutical compositions can be administered according to a pre-determined dosing regimen. Especially, the composition can be administered a specific number of times during each week of treatment. For example, the pharmaceutical composition can be administered three times per week.

Preferably, the formulation is administered daily. Even more preferred, the composition is administered twice daily.

### EXAMPLES:

### Example 1:

### 1.1 Step 1: Preparation of liposomal solution of cyclosporine A

1.1.1 Approximately 70% (~104 L) water for injections was filled into the preparation vessel. It was degassed by introduction of nitrogen gas and warmed up to a temperature of 40 to 45 °C. 18.0 kg of saccharose, 450.0 g of sodium dihydrogen phosphate dihydrate, 612.0 g of disodium hydrogen phosphate decahydrate and 36.0 g of disodium edetate were added together and approximately 5% (8,0 L) of water for injections were used for rinsing. The mixture was stirred until a visually clear solution was obtained. The solution was cooled down to 20 to 25 °C and 6480.0 g of soy bean lecithin S100 was added and stirred until a homogenous dispersion was obtained. Then, 504.0 g of polysorbate 80 HP (Tween 80) was added under gentle stirring to avoid foaming and the container holding the polysorbate was rinsed with approximately 100 mL of water for injections. After that, 720.0 g of cyclosporine A and approximately 5% (8 L) of water for injections was added and the mixture was stirred until a homogenous dispersion was formed.

1.1.2 Following that, the resulting dispersion was cooled to a temperature of 5 to 10 °C and exposed to high pressure homogenization at a pressure of 100 bar (first stage) and 1000 bar (second stage), respectively, using a GEA high pressure homogenizer. The high-pressure homogenization was repeated 9 times (cycles).

1.1.3 The resulting homogenized suspension was then filtered through a bioburden reduction filter with a pore size of 0.2 µm in minimum once and transferred into a filling/storage tank.

### 1.2 Step 2: Aseptic Filling, lyophilization and packaging

1.2.1 Glass vials with a filling volume of 10 mL were sterilized in a hot-air sterilizing tunnel, cooled down and filled with aliquots of 1.35 mL (5mg dosage) of the dispersion as prepared according to step 1 as described above after aseptic sterilisation using 2 sterile filters with a pore size of 0.2 µm between the filling/storage tank and the filling needles. The vials were then partially closed with sterilized lyophilization stoppers and loaded into a lyophilizer, i.e. a GEA Lyovac FCM and were lyophilized according to a 72 h lyophilization cycle.

1.2.2 After completion of lyophilization, the vials were automatically fully stoppered in the lyophilization chamber. The vials were unloaded and closed with flip-tear-off caps. Each vial contained approximately 190 mg of an almost white, homogenous, porous lyophilization cake containing 5 mg of cyclosporine A in liposomally solubilized form with a maximum residual moisture of 2 % (w/w) and a shelf life of 3 years.

1.2.3 The composition of the lyophilized drug product prepared as described above is summarized in Table 1 below:

**Table 1:**

| **Ingredient** | **Quantity per unit** | **Quantity % (w/w)** |
|---|---|---|
| Cyclosporine A | 5 mg | 2.69 |
| Polysorbate 80 | 3.5 mg | 1.88 |
| Lipoid S100 | 45 mg | 24.18 |
| Sucrose | 125 mg | 67.16 |
| Sodium dihydrogen phosphate dihydrate | 3.125 mg | 1. 68 |
| Disodium hydrogen phosphate dodecahydrate | 4.25 mg | 2.28 |
| Disodium edetate dihydrate | 0.25 mg | 0.13 |
| Total | 186.125 mg | 100.00 |

### Example 2: Reconstitution of the lyophilized composition comprising cyclosporine A to yield a colloidal solution of liposomally solubilized cyclosporine A for nebulization and inhalation

2.1 For the preparation of a colloidal solution with a content of liposomally solubilized cyclosporine A of 10 mg, an aliquot of 372.3 mg of the lyophilization cake as prepared according to Example 1 above was dissolved in 2.65 mL of a sterile aqueous sodium chloride solution with a concentration of 0.25 % (w/v) to give an opalescent liquid formulation of liposomal cyclosporine A for inhalation purposes with a concentration of CsA of 4 mg/mL.

2.2 The composition of the reconstituted drug product prepared as described above is summarized in Table 2 below:

**Table 2:**

| **Ingredient** | **Quantity per unit** | **Quantity % (w/v)** |
|---|---|---|
| Cyclosporine A | 10 mg | 0.4 |
| Polysorbate 80 | 7.5 mg | 0.28 |
| Lipoid S100 | 90 mg | 3.6 |
| Sucrose | 250 mg | 10 |
| Sodium dihydrogen phosphate dihydrate | 6.25 mg | 0.25 |
| Disodium hydrogen phosphate dodecahydrate | 8.5 mg | 0.34 |
| Disodium edetate dihydrate | 0.5 mg | 0.02 |
| Sodium chloride | 5.6 mg | 0.22 or 0.23 |
| Water for Injection | Ad 2.5 mL | Ad 100 |

### Example 3:

3.1 The breath simulation experiments were conducted according to Eur. Ph. 2.9.44 using a Compas 2 breath simulator (PARI GmbH, Germany) with a breathing pattern of 500 mL tidal volume at a frequency of 15 breaths/min and an inhalation/exhalation ratio of 50:50.

3.2 2.4 mL of the composition as described in Example 2.2 above were filled into an electronic vibrating membrane nebulizer having a membrane adapted to produce an aerosol with droplets having a mass median aerodynamic diameter (MMAD) in the range of 2.4 to 4.0 µm when measured with a 0.9% (w/v) solution of aqueous sodium chloride. The nebulizer further had a mixing chamber with a volume of 94 mL. The nebulizer was connected to a sinus pump of the breath simulator. The drug containing aerosol droplets were collected on 2 consecutive inspiratory filters (polypropylene filter pad G300, PARI in filter casing with a diameter of 6.5 cm). Between the inspiratory filter and the breath simulator a further filter was installed (BB50 TE, Pall Filtersystems GmbH, Germany). 3.3

After complete nebulization, the inhalation filters were removed, extracted and the extracts analyzed.

3.4 The results of breath simulation experiments as described above are summarized in Table 3 below:

**Table 3:**

| | Mean value | Standard deviation (SD) |
|---|---|---|
| Total Output Rate (TOR) [mg/min] | 245 | 31 |
| Delivered Dose (DD) [mg] | 6.916 | 0.322 |
| Delivered Dose (DD) [%] | 72.0 | 3.4 |
| Exhaled amount [mg] | 0.899 | 0.271 |
| Exhaled amount [%] | 9.4 | 2.8 |
| Residue in nebulizer [mg] | 1.253 | 0.379 |
| Residue in nebulizer [%] | 13.1 | 4.0 |
| Respirable dose (RD ≤ 5µm) [mg] | 5.620 | 0.465 |
| Respirable dose (RD ≤ 5µm) [%] | 58.5 | 4.8 |
| End of aerosol production [min] | 9.64 | 1.62 |
| Automated shutoff [min] | 9.98 | 1.58 |

### Example 4:

4.1 In addition to this, the generated aerosol was characterized according to USP chapter <1601> resp. Ph. Eur. 2.9.44 using the Next Generation Impactor (Next Generation Cascade Impactor, NGI) to assess the aerodynamic droplet size distribution of the nebulized aerosols at an airflow of 15.0 +/- 0.7 L/min, an air temperature of 23.0 +/- 2.0°C and a relative humidity of 50.0 +/- 5.0%. The fill volume was 2.4 mL of the composition as described in Example 2.2. Deviating from the USP procedure the impactor temperature was adapted to the aerosol temperature (18.0 +/- 1.0°C). The nebulizers with the mouthpieces attached were connected via a rubber connector to the induction port of the NGI. The nebulization was conducted and operated until the automatic shutoff of the nebulizer.

4.2 The results of the aerosol characterization experiments (n=5) as described above are summarized in Table 4 below:

**Table 4:**

| | Mean value | Standard deviation (SD) |
|---|---|---|
| Mass median aerodynamic diameter (MMAD) [µm] | 3.26 | 0.24 |
| Geometric Standard Deviation (GSD) | 1.62 | 0.04 |
| Fine Particle Dose (FPD ≤ 5 µm) [mg] | 6.951 | 0.475 |
| Fine Particle Fraction (FPF ≤ 5 µm) [%] | 81.2 | 4.7 |
| End of aerosol production [min] | 10.1 | 1.18 |
| Automated shutoff [min] | 10.41 | 1.25 |

### Comparative Example 1:

Examples 3 and 4 were repeated using an eFlow^{®} Rapid electronic nebulizer (PARI GmbH, Germany) having a vibratable membrane adapted to produce an aerosol with particle having a mass median aerodynamic diameter of 4.1 µm when measured with a 0.9% (w/v) solution of aqueous sodium chloride. The nebulizer further had a mixing chamber with a volume of 48 mL. The results are summarized in Table 5 below.

**Table 5:**

| | Mean value | Standard deviation (SD) |
|---|---|---|
| Total Output Rate (TOR) [mg/min] | 192 | 17 |
| Mass median aerodynamic diameter (MMAD) [µm] | 3.25 | 0.15 |
| Geometric Standard Deviation (GSD) | 1.54 | 0.02 |
| Fine Particle Fraction (FPF ≤ 5 µm) [%] | 86.7 | 2.2 |
| Delivered Dose (DD) [mg] | 3.649 | 0.253 |
| Delivered Dose (DD) [%] | 36.5 | 2.5 |
| Exhaled amount [mg] | 1.287 | 0.486 |
| Exhaled amount [%] | 12.9 | 4.9 |
| Residue in nebulizer [mg] | 4.704 | 0.407 |
| Residue in nebulizer [%] | 47.0 | 4.1 |
| Respirable dose (RD ≤ 5µm) [mg] | 3.161 | 0.182 |
| Respirable dose (RD ≤ 5µm) [%] | 31.6 | 1.8 |
| End of aerosol production [min] | 6.80 | 0.54 |
| Automated shutoff [min] | 7.01 | 0.60 |

## Claims

1. Mouthpiece (40) for delivering an aerosol supplied by a nebulizer (100) to a user, the mouthpiece (40) comprising:
a body (46) defining a fluid path (47) from an inlet port (41) connectable to the nebulizer (100) to an inhalation opening (42) to be received in the mouth of a user; and
a filter (30) having a filter base (31) in fluid communication with the fluid path(47), a filter top (33) detachably connected to the filter base (31) and a filter material (32) provided between the filter base (31) and the filter top (33),
wherein the filter base (31) extends from the body (46) in a direction along a minor axis perpendicular to a major axis of the inhalation opening (42),
the filter top (33) has an exhalation opening (36) cooperating with a one-way valve (39) allowing exhaustion of fluid from the fluid path (47) through the filter material (32) to the outside of the mouthpiece upon exhalation of a patient through the inhalation opening (42); and
the body (46) and the filter base (31) are an integrated one-piece unit,
wherein
a maximum height (H) of the mouthpiece (40) as seen in a side view and seen perpendicular to the minor axis is not more than 90 mm, preferably not more than 85 mm.

2. Mouthpiece according to claim 1, wherein the integrated one-piece unit is an injection molded part.

3. Mouthpiece according to claim 1 or 2, wherein a distance (D) between the filter base (31) and the inhalation opening (42) as seen in a side view is at least 30 mm, preferably at least 35 mm and not more than 50mm, preferably not more than 40 mm.

4. Mouthpiece according to anyone of the preceding claims, wherein a retaining rib (45) for being engaged behind the teeth of a user is provided on an upper side of the body (46) and/or on a lower side of the body (46) adjacent to the inhalation opening (42).

5. Mouthpiece according to anyone of the preceding claims, wherein a front edge (51) of the body (46) surrounding the inhalation opening (42) is curved in a plan view and/or side view.

6. Mouthpiece according to anyone of the preceding claims, wherein the inlet port (41) is conical to be force-fittingly connectable to a boss (106) of the nebulizer (100).

7. Mouthpiece according to anyone of the preceding claims, wherein the inhalation opening (42) is oval-shaped in a front view, the oval shape having the minor axis and the major axis.

8. Nebulizer (100) for delivering an aerosol to a patient comprising:
an aerosol generator (101);
a chamber (105) for temporarily accommodating the aerosol generated by the aerosol generator (101); and
a mouthpiece (40) according to anyone of claims 1 to 6.

9. Nebulizer according to claim 8, wherein the aerosol generator (101), the chamber (105) and the mouthpiece (40) are arranged in that order along the longitudinal direction of the nebulizer (100).

10. Nebulizer according to claim 8 or 9, further comprising a fluid reservoir (103) or an interface configured to connect a fluid reservoir, wherein the aerosol generator (101) comprises a vibratable membrane (110) having a plurality of apertures and separating the fluid reservoir (103) and the chamber (105).

11. Nebulizer according to claim 10, wherein the fluid reservoir (103) or the interface and the membrane (110) are arranged in that order along the longitudinal direction of the nebulizer (100).

12. Nebulizer according to anyone of claims 8 to 10, wherein the chamber (105) has a length (L_{C}) along the longitudinal direction of the nebulizer between 20 mm and 100 mm, particularly 50 mm to 100 mm.

13. Nebulizer according to anyone of claims 8 to 12, wherein the chamber (105) cooperates with an ambient opening (113) having a one-way valve (114) allowing ambient air to enter the chamber (105) from outside of the nebulizer (100) upon inhalation of a patient through the inhalation opening (42) of the mouthpiece (40).

## Patentansprüche

1. Mundstück (40) zum Abgeben eines Aerosols, das von einem Zerstäuber (100) an einen Benutzer geliefert wird, wobei das Mundstück (40) Folgendes umfasst:
einen Körper (46), der einen Fluidpfad (47) von einem Einlassanschluss (41), der mit dem Zerstäuber (100) verbunden werden kann, zu einer Einatmungsöffnung (42), die im Munde eines Benutzers aufgenommen werden soll, definiert; und
einen Filter (30), der eine Filterbasis (31) in fluidischer Kommunikation mit dem Fluidpfad(47), ein Filteroberteil (33), das abnehmbar mit der Filterbasis (31) verbunden ist, und ein Filtermaterial (32), das zwischen der Filterbasis (31) und dem Filteroberteil (33) bereitgestellt ist, aufweist,
wobei sich die Filterbasis (31) von dem Körper (46) in einer Richtung entlang einer kleineren Achse rechtwinkelig zu einer größeren Achse der Einatmungsöffnung (42) erstreckt,
das Filteroberteil (33) eine Ausatmungsöffnung (36) aufweist, die mit einem Rückschlagventil (39) kooperiert, indem sie Ausströmen von Fluid aus dem Fluidpfad (47) durch das Filtermaterial (32) zur Außenseite des Mundstücks bei Ausatmung eines Patienten durch die Einatmungsöffnung (42) zulässt; und
der Körper (46) und die Filterbasis (31) eine integrierte einstückige Einheit sind,
wobei
eine maximale Höhe (H) des Mundstücks (40) in einer Seitenansicht gesehen und rechtwinkelig zu der kleineren Achse betrachtet nicht mehr als 90 mm, vorzugsweise nicht mehr als 85 mm beträgt.

2. Mundstück nach Anspruch 1, wobei die integrierte einstückige Einheit ein Injektionsformteil ist.

3. Mundstück nach Anspruch 1 oder 2, wobei ein Abstand (D) zwischen der Filterbasis (31) und der Einatmungsöffnung (42) in einer Seitenansicht gesehen mindestens 30 mm, vorzugsweise mindestens 35 mm und nicht mehr als 50 mm, vorzugsweise nicht mehr als 40 mm beträgt.

4. Mundstück nach einem der vorstehenden Ansprüche, wobei eine Halterippe (45), die hinter den Zähnen eines Benutzers eingreifen soll, an einer oberen Seite des Körpers (46) und/oder an einer unteren Seite des Körper (46) angrenzend zu der Einatmungsöffnung (42) bereitgestellt ist.

5. Mundstück nach einem der vorstehenden Ansprüche, wobei eine Vorderkante (51) des Körpers (46), die die Einatmungsöffnung (42) umgibt, in einer Draufsicht und/oder Seitenansicht gekrümmt ist.

6. Mundstück nach einem der vorstehenden Ansprüche, wobei der Einlassanschluss (41) kegelförmig ist, um kraftschlüssig mit einem Höcker (106) des Zerstäubers (100) verbindbar zu sein.

7. Mundstück nach einem der vorstehenden Ansprüche, wobei die Einatmungsöffnung (42) in einer Vorderansicht ovalförmig ist, wobei die Ovalform die kleinere Achse und die größere Achse aufweist.

8. Zerstäuber (100) zum Abgeben eines Aerosols an einen Patienten, umfassend:
einen Aerosolgenerator (101);
eine Kammer (105) zum vorübergehenden Aufnehmen des durch den Aerosolgenerator (101) erzeugten Aerosols; und
ein Mundstück (40) nach einem der Ansprüche 1 bis 6.

9. Zerstäuber nach Anspruch 8, wobei der Aerosolgenerator (101), die Kammer (105) und das Mundstück (40) in dieser Reihenfolge entlang der Längsrichtung des Zerstäubers (100) angeordnet sind.

10. Zerstäuber nach Anspruch 8 oder 9, weiter umfassend einen Fluidbehälter (103) oder eine Schnittstelle, die so konfiguriert ist, dass sie einen Fluidbehälter verbindet, wobei der Aerosolgenerator (101) eine schwingfähige Membran (110) umfasst, die eine Vielzahl von Öffnungen aufweist und den Fluidbehälter (103) und die Kammer (105) trennt.

11. Zerstäuber nach Anspruch 10, wobei der Fluidbehälter (103) oder die Schnittstelle und die Membran (110) in dieser Reihenfolge entlang der Längsachse des Zerstäubers (100) angeordnet sind.

12. Zerstäuber nach einem der Ansprüche 8 bis 10, wobei die Kammer (105) eine Länge (L_{c}) entlang der Längsrichtung des Zerstäubers zwischen 20 mm und 100 mm, insbesondere 50 mm bis 100 mm aufweist.

13. Zerstäuber nach einem der Ansprüche 8 bis 12, wobei die Kammer (105) mit einer Umgebungsöffnung (113) kooperiert, die ein Rückschlagventil (114) aufweist, das zulässt, dass beim Einatmen eines Patienten durch die Einatmungsöffnung (42) des Mundstücks (40) Umgebungsluft von außerhalb des Zerstäuber (100) in die Kammer (105) eintritt.

## Revendications

1. Embout buccal (40) destiné à administrer un aérosol fourni par un nébuliseur (100) à un utilisateur, l'embout buccal (40) comprenant :
un corps (46) définissant un trajet de liquide (47) depuis un orifice d'entrée (41) pouvant être relié au nébuliseur (100) jusqu'à une ouverture d'inhalation (42) qui doit être reçue dans la bouche d'un utilisateur ; et
un filtre (30) présentant une base de filtre (31) en communication fluidique avec le trajet de liquide (47), un sommet de filtre (33) relié de manière détachable à la base de filtre (31) et un matériau de filtre (32) prévu entre la base de filtre (31) et le sommet de filtre (33),
dans lequel la base de filtre (31) s'étend depuis le corps (46) dans une direction le long d'un axe mineur perpendiculaire à un axe majeur de l'ouverture d'inhalation (42),
le sommet de filtre (33) présente une ouverture d'expiration (36) coopérant avec une valve unidirectionnelle (39) permettant l'échappement de liquide depuis le trajet de liquide (47) à travers le matériau de filtre (32) vers l'extérieur de l'embout buccal lors de l'expiration d'un patient à travers l'ouverture d'inhalation (42) ; et
le corps (46) et la base de filtre (31) se trouvent dans une unité monobloc intégrée,
dans lequel
une hauteur maximale (H) de l'embout buccal (40) telle que vue dans la vue de côté et perpendiculairement à l'axe mineur n'est pas supérieure à 90 mm, de préférence non supérieure à 85 mm.

2. Embout buccal selon la revendication 1, dans lequel l'unité monobloc intégrée est une pièce moulée par injection.

3. Embout buccal selon la revendication 1 ou 2, dans lequel une distance (D) entre la base de filtre (31) et l'ouverture d'inhalation (42) telle que vue dans la vue de côté est au moins de 30 mm, de préférence au moins de 35 mm et non supérieure à 50 mm, de préférence non supérieure à 40 mm.

4. Embout buccal selon l'une quelconque des revendications précédentes, dans lequel une nervure de retenue (45) destinée à être engagée derrière les dents d'un utilisateur est prévue sur un côté supérieur du corps (46) et/ou sur un côté inférieur du corps (46) de manière adjacente à l'ouverture d'inhalation (42).

5. Embout buccal selon l'une quelconque des revendications précédentes, dans lequel un bord avant (51) du corps (46) entourant l'ouverture d'inhalation (42) est incurvé dans une vue en plan et/ou une vue de côté.

6. Embout buccal selon l'une quelconque des revendications précédentes, dans lequel l'orifice d'entrée (41) est conique pour pouvoir être relié par ajustement forcé à un bossage (106) du nébuliseur (100).

7. Embout buccal selon l'une quelconque des revendications précédentes, dans lequel l'ouverture d'inhalation (42) est de forme ovale dans une vue de face, la forme ovale présentant l'axe mineur et l'axe majeur.

8. Nébuliseur (100) destiné à administrer un aérosol à un patient comprenant :
un générateur d'aérosol (101) ;
une chambre (105) destinée à loger temporairement l'aérosol généré par le générateur d'aérosol (101) ; et
un embout buccal (40) selon l'une quelconque des revendications 1 à 6.

9. Nébuliseur selon la revendication 8, dans lequel le générateur d'aérosol (101), la chambre (105) et l'embout buccal (40) sont agencés dans cet ordre le long de la direction longitudinale du nébuliseur (100).

10. Nébuliseur selon la revendication 8 ou 9, comprenant en outre un réservoir de fluide (103) ou une interface configurée pour relier un réservoir de fluide, dans lequel le générateur d'aérosol (101) comprend une membrane vibrante (110) présentant une pluralité de lumières et séparant le réservoir de fluide (103) et la chambre (105).

11. Nébuliseur selon la revendication 10, dans lequel le réservoir de fluide (103) ou l'interface et la membrane (110) sont agencés dans cet ordre le long de la direction longitudinale du nébuliseur (100).

12. Nébuliseur selon l'une quelconque des revendications 8 à 10, dans lequel la chambre (105) a une longueur (L_{c}) le long de la direction longitudinale du nébuliseur comprise entre 20 mm et 100 mm, en particulier de 50 mm à 100 mm.

13. Nébuliseur selon l'une quelconque des revendications 8 à 12, dans lequel la chambre (105) coopère avec une ouverture ambiante (113) présentant une valve unidirectionnelle (114) permettant à l'air ambiant d'entrer dans la chambre (105) depuis l'extérieur du nébuliseur (100) lors de l'inhalation d'un patient à travers l'ouverture d'inhalation (42) de l'embout buccal (40).
